Europäisches Patentamt

(19) European Patent Office

Office européen des brevets

(11) Publication number: **0 208 418 B1**

# EUROPEAN PATENT SPECIFICATION

(45) Date of publication of patent specification: **30.10.91**

(51) Int. Cl.5: **C12N 15/29, C07K 13/00, A01H 1/00, C12P 21/02, C12N 1/20, C12N 5/00**

(21) Application number: **86304370.9**

(22) Date of filing: **09.06.86**

(54) **Modified zein.**

(30) Priority: **12.06.85 US 744913**

(43) Date of publication of application:
**14.01.87 Bulletin 87/03**

(45) Publication of the grant of the patent:
**30.10.91 Bulletin 91/44**

(84) Designated Contracting States:
**AT BE CH DE FR GB IT LI LU NL SE**

(56) References cited:
**WO-A-83/01176**

**CHEMICAL ABSTRACTS, vol. 95, 1981, page 397, abstract no. 200780c, Columbus, Ohio, US; D. KALINNIKOV et al.: "Lysine and tryptophan content of zein from corn mutants produced by laser irradiation"**

**FEDERATION PROCEEDINGS, 69TH ANNUAL MEETING, Anaheim, Calfornia, 21st-26th April 1985, vol. 44, no. 3, 1st March 1985, page 649, no. 1396; B.A. LARKINS et al.: "Genetic engineering of maize zein proteins"**

(73) Proprietor: **LUBRIZOL GENETICS INC.**
**3375 Mitchell Lane**
**Boulder Colorado 80301-2244(US)**

Proprietor: **PURDUE RESEARCH FOUNDATION, INC.**
**Frederick L. Hovde Hall of Administration**
**West Lafayette, Indiana 47907(US)**

(72) Inventor: **Larkins, Brian**
**2553 Shagbark Lane**
**West Lafayette Indiana 47906(US)**
Inventor: **Cuellar, Richard**
**615 Dodge Street**
**West Lafayette Indiana 47907(US)**

(74) Representative: **Fisher, Adrian John et al**
**CARPMAELS & RANSFORD 43 Bloomsbury Square**
**London WC1A 2RA(GB)**

GENETICA AGRARIA: A JOURNAL DEVOTED TO AGRICULTURAL GENETICS, vol. 40, no. 4, 1986, Italian Society for Agricultural Genetics - Meeting 1985, pages 443-444, Istituto Biosintesi Vegetali C.N.R., Milan, IT; I. CORAGGIO et al.: "Expression of normal and modified zein sequences in yeast"

GENETICA AGRARIA: A JOURNAL DEVOTED TO AGRICULTURAL GENETICS, vol. 40, no. 4, 1986, Italian Society for Agricultural Genetics - Meeting 1985, pages 443-444, I. CORAGGIO et al.: "Site directed mutagenis of zein genes"

GENETICA AGRARIA: A JOURNAL DEVOTED TO AGRICULTURAL GENETICS, vol. 39, no. 3, 1985, Italian Society for Agricultural Genetics - Meeting 1984, page 318, Istituto Biosintesi Vegetali C.N.R., Milan, IT; I. CORAGGIO et al.: "Insertion of zein sequence in interspecific Escherichia-coli and saccharomyces-cerevisiae vectors and studies on their expression in yeast"

THE EMBO JOURNAL, vol. 4, no. 5, 1985, pages 1103-1110, IRL Press Ltd, Oxford, GB; A. VIOTTI et al.: "Each zein gene class can produce polypeptides of different sizes"

DISSERTATION ABSTRACTS INTERNATIONAL, vol. 45, no. 12, June 1985, J.M. NORRANDER; "Zein storage proteins expressed from synthetic genes altered by oligonucleotide-directed in vitro mutagenesis"

JOURNAL OF BIOTECHNOLOGY, vol. 2, 1985, pages 157-175, Elsevier Science Publishers B.V.; J.M. NORRANDER et al.: "Manipulation and expression of the maize zein storage proteins in Escherichia coli"

JOURNAL OF CELL BIOLOGY, vol. 89, May 1981, pages 292-299, The Rockefeller University Press, US; W.J. HURKMAN et al.: "Subcellular compartmentalization of maize storage proteins in Xenopus Oocytes injected with zein messenger RNAs"

TRENDS IN BIOTECHNOLOGY, vol. 1, no. 2, 1983, pages 54-59, Elsevier Science Publishers B.V., Amsterdam, NL; J. MESSING: "The manipulation of zein genes to improve the nutritional value of corn"

PROCEEDINGS OF THE NATIONAL ACADEMY OF SCIENCES OF USA, vol. 76, no. 12, December 1979, pages 6448-6452; B.A. LARKIN et al.: "Synthesis and processing of maize storage proteins in Xenopus laevis oocytes"

EUROPEAN JOURNAL OF CELLULAR BIOLOGY, vol. 33, no suppl. 5, 1984, page 26; G. NEUHAUS et al.: "Expression of zein genes in Acetabularia"

JOURNAL OF CELLULAR BIOCHEMISTRY, vol. 0, np. 10, part D, 1986, page 53, abstract no. N137; D. DONALDSON et al.: "Expression of seed storage proteins in xenopus oocytes"

THE EMBO JOURNAL, vol. 3, no. 7, 1984, pages 1525-1531, IRL Press Ltd, Oxford, GB; M.A. MATZKE et al.: "Transcription of a zein gene introduced into sunflower using a Ti plasmid vector"

CELL, vol. 29, July 1982, July 1982, pages 1015-1026, MIT; K. PEDERSEN et al.: "Cloning and sequence analysis reveal structural variation among related zein genes in maize"

SCIENCE, vol. 219, 11th February 1983, pages 671-676; K.A. BARTON et al.: "Prospects in plant genetic engineering"

JOURNAL OF BIOLOGICAL CHEMISTRY, vol. 257, no. 17, 10th September 1982, pages 9976-9983, US; M.D. MARKS et al.: "Analysis of sequence microheterogeneity among zein messenger RNAs"

PROCEEDINGS OF THE NATIONAL ACADEMY OF SCIENCES OF USA, vol. 82, May 1985, pages 3320-3324; C. SENGUPTA-GOPALAN et al.: "Developmentally regulated expression of the bean beta-phaseolin gene in tobacco seed"

## Description

Seed storage proteins are the major source of protein in a vegetarian diet. Most seed storage proteins are nutritionally incomplete in that they lack one or more of the essential amino acids for proper nutrition of higher animals including man. Reliance on a single source of vegetable protein to meet dietary needs leads to deficiency conditions. For example, the cereal seed storage proteins typically lack the essential amino acid lysine, and overdependence on a cereal, notably corn, for dietary protein is in part responsible for the condition of malnutrition known as kwashiorkor.

When using corn as feed or in the human diet, it is therefore necessary to supplement the diet with another protein source or with lysine itself. Such supplemental feeding is not always convenient or economically feasible. The present invention provides a modified zein which contains lysine. The modified zein serves as a nutritionally balanced source of protein. The modified zein can be provided in any form acceptable to the intended consumer, e.g., as a single cell protein, or as the seed storage protein of a genetically modified plant, which may be maize, soybean, sunflower or other plant species carrying and expressing a gene encoding the modified zein, either in its seeds or other edible tissues.

Maize Zein Proteins

The storage proteins of maize seed consist of a group of prolamine proteins called zeins. (For review, see Larkins, B., Genetic Engineering of Plants (T. Kosuge, C. P. Meredith and A. Hollander eds.) Plenum, New York 1983, pp. 93-118.) The zeins are water-insoluble, but soluble in alcohol. Zein proteins are synthesized by membrane-bound polyribosomes in the developing endosperm and are deposited as aggregates called protein bodies within the rough endoplasmic reticulum (RER). Because of this, one observes RER membranes surrounding protein bodies in electron micrographs of maize endosperm. It is often difficult to observe continuity between membranes surrounding the protein bodies and the RER, but previous studies have shown the existence of similar populations of polyribosomes on the surface of both membranes (Larkins, B. A. and W. J. Hurkman (1978) Plant Physiol. 62:256-263). Perhaps the most convincing evidence that zein protein bodies form simply by protein aggregation within the RER is the observation that structures with the same physical characteristics as protein bodies can be isolated from Xenopus laevis oocytes previously injected with zein mRNAs (Hurkman et al. (1981) J. Cell. Biol. 89:292-299).

A 2-dimensional gel analysis of the alcohol-soluble proteins contained within the protein bodies reveals a mixture of polypeptides. The most abundant of these have apparent molecular weights of 22,000 and 19,000, but there are also some smaller polypeptides of 15,000 and 10,000 daltons. There is significantly more charge heterogeneity among the Mr 22,000 and Mr 19,000 zeins than the small molecular weight zein proteins.

Marks and Larkins (1982) J. Biol. Chem. 257:9976-9983, and Pederson, et al. (1982) Cell 29:1015-1026 have constructed cDNA clones of zein mRNAs and determined the DNA sequence for representative Mr 22,000, Mr 19,000, and Mr 15,000 zein proteins. The sequence for an Mr 19,000 zein has also been reported by Geraghty et al. (1981) Nucleic Acids Res. 9:5163-5174. From knowledge of the DNA sequence it has been possible to determine the complete primary amino acid sequence of the polypeptides and compare them for structural similarities.

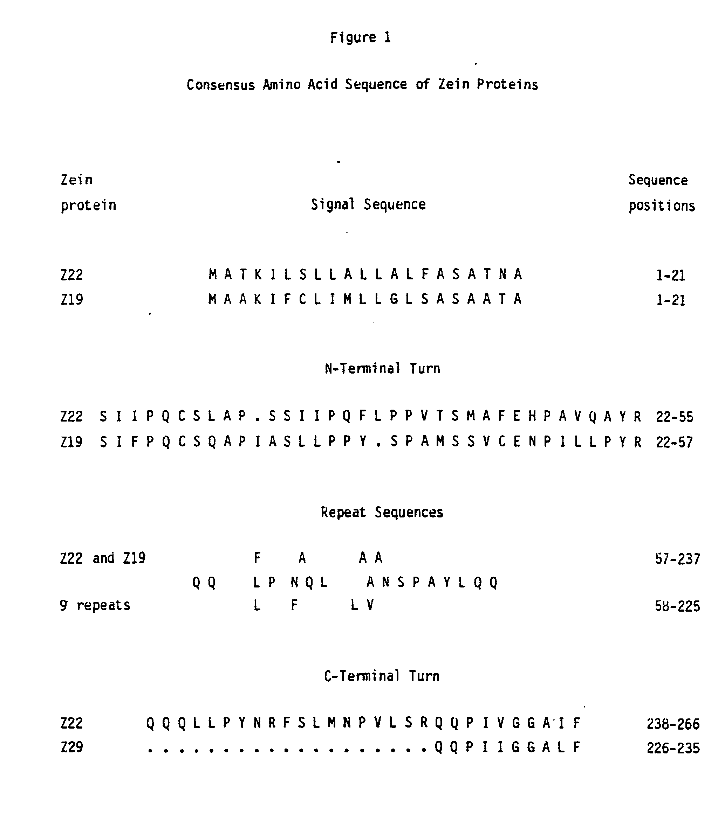

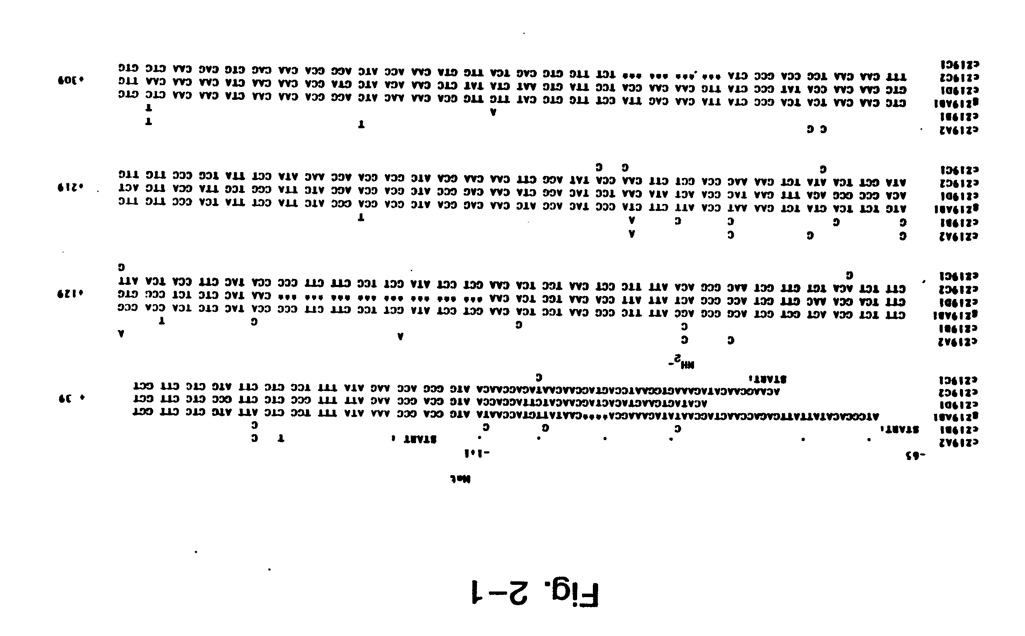

This analysis revealed that the zeins were significantly larger than expected based on their mobility on SDS polyacrylamide gels. Zein proteins that had been estimated to have molecular weights of 22,000 and 19,000 were found to be closer to 27,000 and 23,000, respectively (Table I). This analysis also confirmed the presence of signal peptides on the zein proteins (Fig. 1). These signal sequences were previously demonstrated to be removed when the protein is transported into the lumen of the RER (Larkins et al. (1979) Proc. Natl. Acad. Sci. USA 76:6448-6452).

TABLE I

Amino Acid Composition of Maize Zein Proteins

| Apparent Molecular Weight | [a]Zein α<br>Mr 22,000 | [b]Zein β<br>Mr 19,000 | Zein<br>Mr 15,000 |
|---|---|---|---|
| Amino Acid | | | |
| Leu | 42 | 44 | 15 |
| Gln | 41 | 39 | 28 |
| Ala | 34 | 31 | 18 |
| Pro | 22 | 21 | 13 |
| Ser | 18 | 15 | 11 |
| Phe | 8 | 13 | 0 |
| Asn | 13 | 9 | 2 |
| Ile | 11 | 9 | 1 |
| Tyr | 6 | 8 | 16 |
| Val | 17 | 6 | 4 |
| Gly | 2 | 4 | 12 |
| Thr | 7 | 4 | 5 |
| Arg | 4 | 3 | 7 |
| His | 3 | 3 | 4 |
| Cys | 1 | 2 | 6 |
| Glu | 1 | 1 | 5 |
| Met | 5 | 1 | 11 |
| Asp | 0 | 1 | 4 |
| Lys | 0 | 0 | 0 |
| Trp | 0 | 0 | 1 |
| TOTAL | 225 | 214 | 163 |

[a] Marks and Larkins (1982) J. Biol. Chem. 257:9976-9983

[b] Pedersen et al. (1982) Cell 29:1015-1026

The amino acid composition predicted from the polypeptide sequence is similar to that previously found for mixtures of zein proteins (Lee et al. (1976) Biochem. Genet. 14:641-650). Glutamine, leucine, proline, alanine, and glycine account for the majority of the amino acids, and lysine and tryptophan absent from all of them (Table I). It is interesting to note that methionine, which is deficient in most legume storage proteins, accounts for a significant percentage of the Mr 15,000 zein. In fact, cysteine and methionine account for 11% of the total amino acids in this polypeptide.

A particularly interesting feature of the protein sequence is the occurrence of a conserved, tandemly repeated peptide in both of the Mr 22,000 and Mr 19,000 zeins (Fig. 1). The first of these repeat sequences begins 35-36 amino acids after the NH2-terminus, and is repeated nine times in each polypeptide. The COOH-terminal sequence following the repeats is slightly longer in the Mr 22,000 zeins; this accounts for the size difference between the two polypeptides. Most of the amino acids in these repeats are nonpolar, while the repeated peptide is sequentially polar, nonpolar, polar, nonpolar, polar.

Circular dichroism measurements of mixtures of zein proteins indicate from 45-55% α-helical structure (Argos et al. (1982) J. Biol. Chem. 257:9984-9990) and this percentage correlates well with the proportion of amino acids in these repeated peptides. To determine if the repeats have the potential to form α-helices we compared their amino acid sequences with those found in proteins having α-helical structure (Argos et al., supra) Although a comparison to soluble proteins shown little propensity for these repetitive sequences to

be α-helical, they do have α-helical properties when compared with sequences found in some hydrophobic proteins. In view of the hydrophobic nature of zein proteins, it seems reasonable to predict an α-helical structure for them.

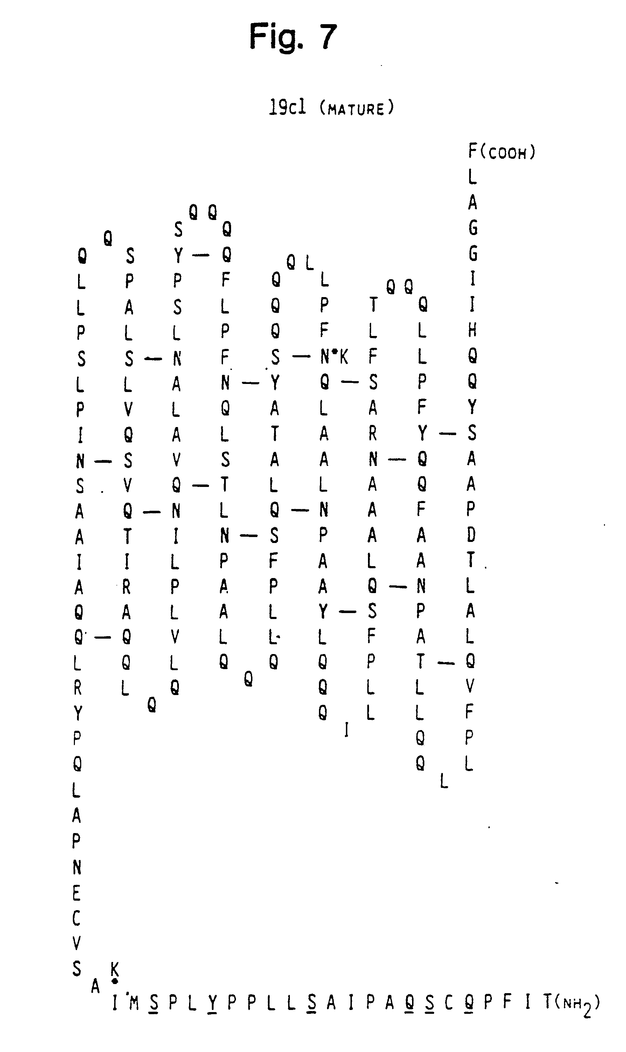

Assuming that these are α-helices, a model showing how the nine repeats could be organized into a 3-dimensional structure has been published. The model predicts that when the consensus repeat is placed in an α-helical wheel the polar amino acids are distributed at three symmetrical sites. Considering that the repeats are tandem, if they fold back upon one another in an anti parallel arrangement, two polar groups in each repeat can hydrogen bond with each of two adjacent repeats. The nine helices would then interact to form a roughly cylindrical, rod shaped molecule. The cylinder would collapse in the center to accommodate the non-polar tails of the amino acids. As these protein molecules associate within the endoplasmic reticulum, the third polar group, which is on the surface of the helix, would hydrogen bond to a different zein molecule. This arrangement also allows the glutamine residues, which lie at the ends of the helices, to hydrogen bond with neighboring protein molecules in the protein body. Fig. 7 provides a two-dimensional representation of the proposed model.

The published model explains many of the physical properties of the proteins (Argos et al., supra), although it has not been confirmed by x-ray diffraction patterns of protein crystals. If the interaction of these α-helices is important in structuring the polypeptide and aggregating it into a protein body, it would appear that altering the amino acid sequence of these repeated regions could deleteriously affect the protein's structure. Larkins (1983) supra, stated that it would seem more advantageous to change the $NH_2$-terminal or COOH-terminal turn sequences which lie outside the repeat structures.

## SUMMARY OF THE INVENTION

The invention includes modified 19 kd and 22 kd zeins containing lysine, modified structural genes encoding protein precursors to said modified zeins and various genetic constructs and vectors comprising these modified genes. The modified zeins resemble normal unmodified zeins in their molecular weights, ability to form protein bodies within the rough endoplasmic reticulum of a host cell, and solubility in alcohol.

## DETAILED DESCRIPTION OF THE INVENTION

Conventional nomenclature for the zeins refers to their nominal molecular weights based on mobility on SDS polyacrylamide gels. Although the actual molecular weights, deduced from the nucleotide sequences of the genes encoding these proteins is somewhat larger, as stated supra, the proteins will be referred to in terms of the conventional nomenclature. Zeins of molecular weight of approximately 15 kd, 19 kd and 22 kd have been reported. The present invention relates to the 19 kd and 22 kd zeins. The secondary and tertiary structures of the 19 kd and 22 kd zeins is very similar, the primary difference being that the larger zeins have an extended COOH-terminal sequence. The principles of amino acid substitution are exemplified herein in connection with a 19 kd zein. Similar modifications can be made in the 22 kd zeins without substantially affecting their zein-like properties.

An important operating principle maintained throughout is that a modification must not interfere with the ability of the modified protein to function as a storage protein in maize endosperm. The unusual solubility properties of zein, the ability to be translocated within the cell and the ability to form characteristic protein bodies are considered important criteria for correct functioning of a modified zein protein. Hurkman et al. (1981) have demonstrated that the foregoing criteria can be measured in the frog oocyte expression system. Thus, authentic pre-zein mRNA can be injected into frog (Xenopus laevis) oocytes wherein translation yields a protein which accumulates in rough endoplasmic reticulum in protein bodies resembling those found in maize endosperm tissue. The term "modified zein" is therefore reserved for zein proteins having an amino acid sequence which is not naturally occurring, which behaves similarly to authentic zein in a frog oocyte expression system and which is soluble in alcohol. The term "pre-zein" denotes the primary translation product of mRNA comprising a zein coding sequence, said primary translation product comprising an additional $NH_2$-terminal sequence not found in mature zein isolated from protein bodies. The additional NH2-terminal sequence is believed to function as a signal peptide which aids in intracellular compartmentalization of the translation product. The terms "modified pre-zein" and "modified pre-zein gene" as used herein have the same meanings with respect to the modified sequences as those used in connection with the unmodified sequences, the only difference being related to changes in the primary amino acid sequences described herein.

An important feature of the present invention is based upon the unexpected finding that the substitution of a lysine within one of the repeated segments of the molecule yields a modified, lysine-containing zein

EP 0 208 418 B1

product that meets the functional and physical criteria described, supra. The invention has been exemplified by the modification of a DNA encoding a 19 kd pre-zein gene such that the coding sequence encodes a lysine in the place of an amino acid of the naturally-occurring sequence.

In the examples, the conversion of an asparagine residue to lysine at a locus within the repeated portion of the sequence yielded a lysine containing modified zein which behaved normally when translated in frog oocytes. On the other hand, conversion of an isoleucine residue to lysine in the NH2 terminal turn of the mature polypeptide did not yield a detectable protein having the properties of a zein as judged by the before-mentioned criteria. The results demonstrate that, contrary to expectation, substitution of a lysine for an amino acid in the repeated portion of the molecule is tolerated without detrimental effect to zein function and properties. It will be understood by those of ordinary skill in the art, following the teachings of the present invention, that modifications other than those exemplified can be used to introduce a desired modification in amino acid composition while retaining the functional and physical properties of zein. Amino acid substitutions other than lysine for asparagine, especially those which do not substantially modify the hydrophobicity of the repeated regions may be introduced. An important operating principle is to preserve the overall hydrophobic character of the internal repeats so as to preserve their character as domains responsible for establishing the tightly folded configuration of the zein molecule. A second principle is to retain the conformation and hydrophobicity of the NH2-terminal sequences of modified pre-zein and zein, in order to retain the function of correct translocation in the host cell. Since lysine is protonated at intracellular pH levels, lysine replacement of a highly non-polar amino acid is likely to be more disruptive than replacement of a polar, or moderately non-polar amino acid. In addition, amino acid residues that are attractive candidates for replacement will be located in less conserved regions of the repeated sequences. Such less conserved (divergent) regions can be identified by comparisons of several 19 kd or 22 kd zein sequences. Besides amino acid substitution within the repeated sections, single amino acid insertions can be employed, as well as substitutions in the segments connecting the domains of repeated sequences. Also, insertions and substitutions can be made in the COOH-end of the zein molecule. In particular, the substitution of a lysine for a glutamine within the segment postulated to connect two repeated segments yields a structure having the required properties. An upper limit to the number of lysines which may be introduced into a modified 19 kd or 22 kd zein has not been determined. A determination of an upper limit is not critical, however, because as little as one lysine residue per modified zein molecule is sufficient to confer a valuable nutritional benefit. Regulation of the amount of lysine produced by cells or tissues expressing a modified zein coding sequence can be achieved in one of two ways: by increasing the number of lysine residues per molecule or by increasing the number of molecules synthesized per cell. The latter can be achieved according to well known principles in the art, using an appropriate promoter whose activity is sufficient to insure the desired level of gene expression in the desired host cell.

Means for modifying a 19 kd or 22 kd zein structural gene are well known in the art and may be employed according to well known and understood principles in the art to achieve the desired modification. The method of site-specific mutagenesis, using M13-based single stranded DNA vectors and synthetic oligonucleotide primers is exemplified herein, but other means for effecting the desired coding sequence changes may be employed. The technique of site-specific mutagenesis employs the chemically synthesized oligonucleotide corresponding to the coding segment whose modification is desired, with a base substitution at the exact site where a change in the coding sequence is desired. The synthetic oligonucleotide is allowed to hybridize with single stranded DNA comprising its complement, then used to prime DNA synthesis of a new coding strand incorporating the desired base change. When the technique is being used to alter a sequence lying within a region known to contain repeats, care must be taken to insure that the oligonucleotide binds to the template at the desired site. The oligonucleotide must be either sufficiently unique or sufficiently long to prevent mispairing.

The DNA encoding native, or unmodified, zein may be cloned as cDNA or genomic DNA. Although maize contains a number of genes encoding both 19 kd and 22 kd zeins, none has been found to date which contain introns. Consequently, the modification of cDNA is an entirely feasible strategy even if the end result desired is to reincorporate the modified coding sequence into the maize genome to produce genetically modified maize containing nutritionally balanced zein.

A variety of systems. for expressing modified zein can be used. A structural gene encoding modified pre-zein may be combined with a promoter known to provide adequate levels of expression in the chosen host cell. The host cells may be any host cell in which expression of modified zein is or can be made compatible, including bacteria, fungi, yeasts, plant cells and animal cells. For example, a modified pre-zein structural gene as exemplified herein can be combined with the phaseolin promoter, which is known to provide tissue-specific expression in plants (Sengupta-Gopalan et al. (1985) Proc. Natl. Acad. Sci. USA, 82:3320-3324. The composite gene thus constructed can be introduced to plant cells of a desired species

7

using any of a variety of vectors comprising the T-DNA of Agrobacterium tumefaciens Ti plasmid (See Caplan, A. et al. (1983) Science 222:815; Barton, K. A. and Chilton, M.-D. (1983) Meth. Enzymol. 101:527; and Leemans, J. et al. (1982) in Molecular Biology of Plant Tumors (G. Kahl and J. Schell, eds.) Academic Press, NY, p 537). Depending upon the means used for introducing the composite gene into plant cells, and the host plant cell species chosen, the cells receiving the composite gene can be regenerated to form fertile adult plants according to known techniques. Seeds of the regenerated plant will express the modified zein thereby providing a nutritionally balanced diet with respect to amino acid composition for humans or animals using the seeds of the plant as food. Current techniques for gene transfer and regeneration are available for such agronomically significant crops as tomato, soybean, sunflower and a number of vegetable crops. Furthermore, the number of crops in which gene transfer and regeneration techniques are being developed is continuously increasing. Expression of a modified zein need not be confined to seed or endosperm tissue. In some instances it will be desirable to enhance the nutritional value of leaves, stems and other edible vegetative tissues of a plant, to enhance the nutritional value of the plant.

In addition to plants, other organisms, especially single celled organisms, may be genetically altered to produce a modified zein, for example as a convenient form of single cell protein. The properties of zein which make it useful for storage of protein in a highly concentrated and stable form in the maize endosperm are expected to be of value for the production of highly nutritious single cell protein.

Modified pre-zein genes may also be expressed in animal cells. In fact, an animal cell system, frog oocytes, was employed as detailed in the examples, for the purpose of rapidly determining whether a given genetic modification yields a modified zein having the appropriate functional and physical characteristics. In the examples, a modified pre-zein coding segment was cloned into the E. coli vector SP6. Recombinant SP6, carrying the inserted pre-zein coding segment, was used as template in an in vitro RNA polymerase-catalyzed reaction to produce messenger RNA encoding modified pre-zein. The RNA preparation was then injected into frog oocytes which, when incubated in an appropriate medium under known techniques and conditions, are capable of translating the injected mRNA. The synthesis of zein or modified zein, as the case may be, was measured by the appearance of a protein band on an electrophoresis gel, after extracting alcohol soluble, aqueous insoluble protein from the oocytes. The appearance of a protein band at a position corresponding to 19 kd or 22 kd, as the case may be, indicated the synthesis of zein, or modified zein by the oocytes. In a second type of assay, the microscopic appearance of protein bodies in the rough endoplasmic reticulum of oocytes was observed in oocytes expressing native zein or zein modified according to the invention.

In the course of the experiments described in the examples, it was observed that cDNA which had originally been cloned using the technique of homopolymer tailing resulted, after transcription using the SP6 system, in messenger RNA which was poorly translated. Translation was substantially enhanced by pretreating the cDNA with limited exonuclease digestion to remove the homopolymer tails, prior to cloning into SP6.

The following examples further illustrate the invention. Except as noted hereafter, standard techniques for cloning, DNA isolation, amplification and purification, for enzymatic reactions involving DNA ligase, DNA polymerase, restriction endonucleases and the like, and various separation techniques are those known and commonly employed by those skilled in the art (See, for example, R. Wu, ed. (1979) Meth. Enzymol. 68; R. Win et al., eds. (1983) Meth. Enzymol. 100, 101: L. Grossman and K. Moldave, eds. (1980) Meth. Enzymol. 65; J. H. Miller (1972) Experiments in Molecular Genetics; R. Davis et al. (1980) Advanced Bacterial Genetics; R. F. Schleif and P. C. Wensink (1982) Practical Methods in Molecular Biology; and T. Manniatis et al. (1982) Molecular Cloning.). Abbreviations, where employed, are those deemed standard in the field and commonly used in professional journals of wide circulation such as those cited herein.

Textual use of the name of a restriction endonuclease in isolation, e.g., "BclI" refers to use of that enzyme in an enzymatic digestion, except in a diagram where it can refer to the site of a sequence susceptible to action of that enzyme, e.g., a restriction site. In the text, restriction sites are indicated by the additional use of the word "site", e.g.; "BclI site". The additional use of the word "fragment", e.g., "BclI fragment", indicates a linear double-stranded DNA molecule having ends generated by action of the named enzyme (e.g., a restriction fragment). A phrase such as BclI/SmaI fragment" indicates that the restriction fragment was generated by the action of two different enzymes. Note that the ends will have the characteristics of being either sticky (i.e., having a single strand of protrusion capable of base-pairing with a complementary single-stranded oligonucleotide) or blunt (i.e., having no single-stranded protrusion) and that the specificity of a sticky end will be determined by the sequence of nucleotides comprising the single-stranded protrusion which in turn is determined by the specificity of the enzyme which produces it.

Example 1: Cloning of double-stranded cDNAs

Zein mRNAs were isolated from membrane-bound polyribosomes of the maize inbred W64A as described by Larkins and Hurkman (1978) and used as templates for double-stranded (ds) cDNA synthesis as described by Buell et al. (1978) J. Biol. Chem. 253:2471-2482. S1 nuclease-treated ds-cDNAs were fractionated on a 5% polyacrylamide slab gel. Gel slices that contained cDNAs of 700 or more bases were placed in dialysis bags filled with 1 ml of 0.5X TBE (TBE = 0.01 M Tris, pH 8.3, 0.01 M boric acid, 0.01 mM EDTA) and were subjected to an electric field of 50 volts for 2 hours. The ds-cDNA was recovered by ethanol precipitation in the presence of 0.3 M Na acetate. Homopolymer tails of oligo (dC) were added to the ds cDNA using terminal transferase as described by Roychoudhury et al. (1976) Nucleic Acids Res. 3:101-116 and the ds cDNA was annealed to the plasmid pUC8 that had been homopolymer tailed with oligo (dG). The recombinant plasmids were used to transform the bacterial strain JM83 that was made competent by the method of Morrison (1979) Meth. Enzymol. 68:326. Bacteria containing recombinant plasmids were identified by the filter hybridization procedure of Grunstein and Hogness (1975) Proc. Natl. Acad. Sci. USA 72:3961-3965 using $^{32}$P-labeled cDNA as a probe. B using then pUC 8-JM83 transformation system (Messing and Vieira (1982) Gene 19:269-276), a 10-fold increase was obtained in cloning efficiency over the pBR 322-HB101 system that was previously used.

The sequences of several 19 kd and 22 kd cDNA clones are shown in Fig. 2 and Fig. 3, respectively. The corresponding amino acid sequences are shown in Figs. 4 and 5.

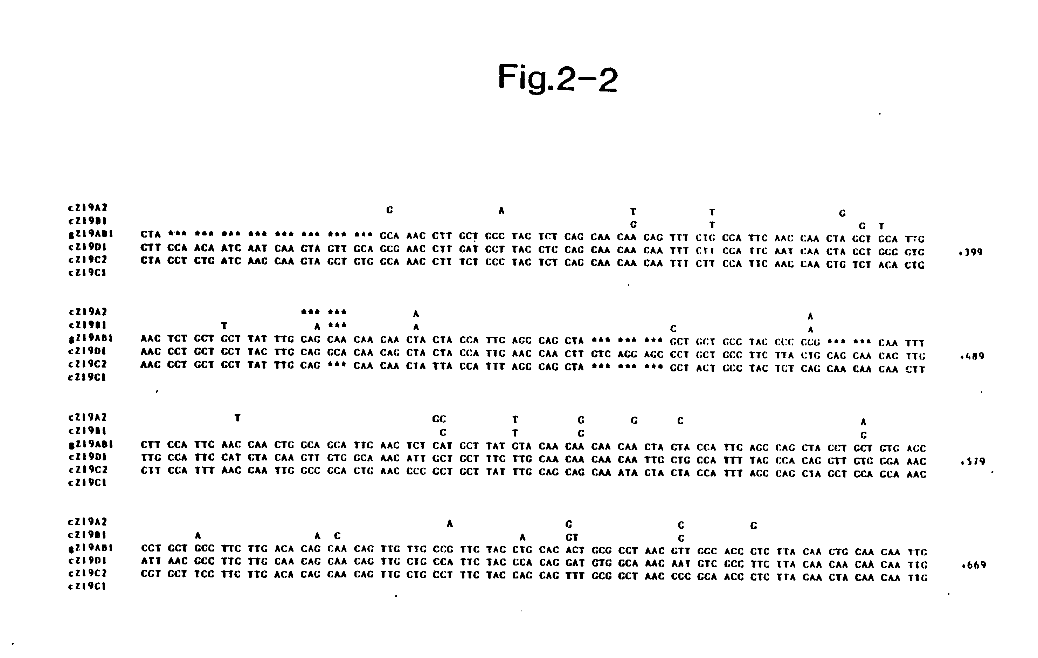

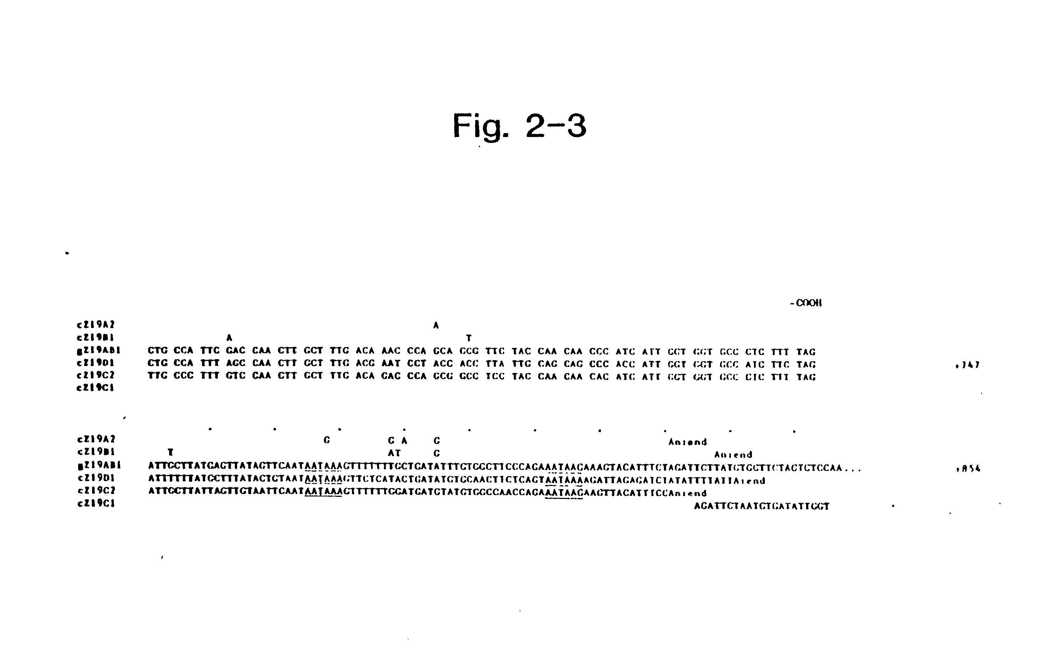

Figure 2 shows the nucleotide sequence of clones for the 19 kd zeins. The nucleotide sequence of gZ19AB1 (previously named ZG99) was previously reported (Pederson et al., 1982). The complete sequences of gZ19AB1, cZ19D1, and cZ19C2 are given. The sequences of the cZ19A2 and cZ19B1 follow the colons and only nucleotides in the sequences that vary from gZ19AB1 are shown. Likewise, the cZ19C1 sequence begins after the colon and only nucleotides in that sequence which vary from cZ19C2 are shown. "An" indicates a sequence terminating in a poly(A) tail. Asterisks indicate positions where gaps were introduced in the sequences to maximize homology. The putative polyadenylation signals are underlined. Positions corresponding to the initiating methionine (Met) and the NH$_2$-terminus and -COOH terminus of the encoded polypeptide are indicated.

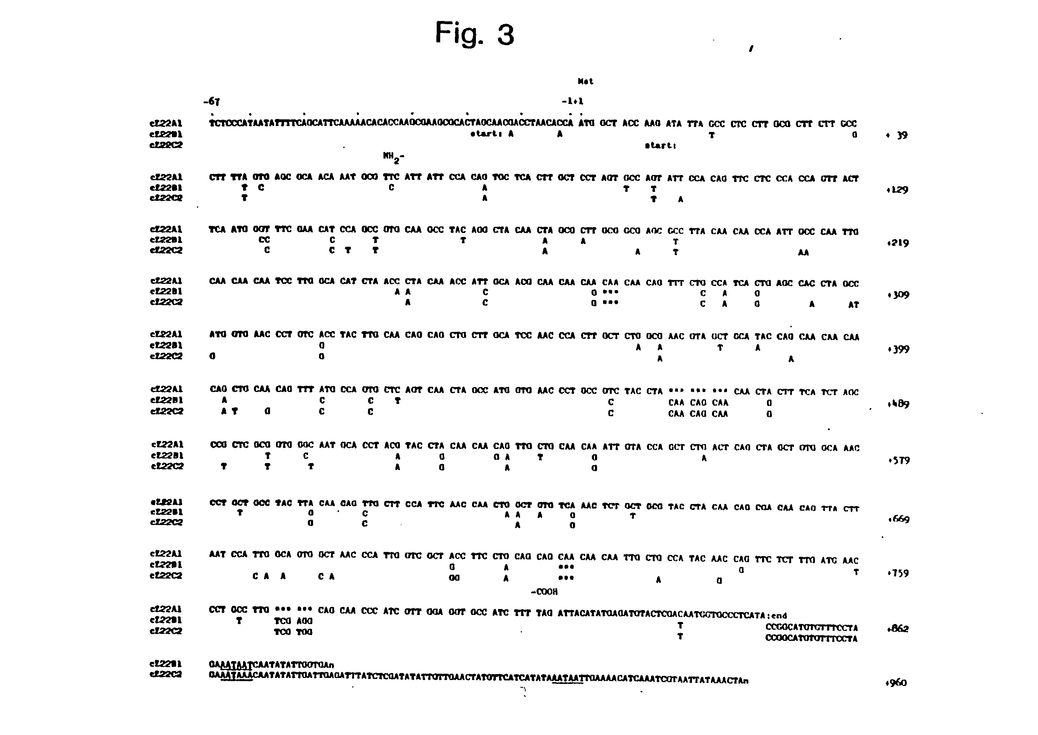

Figure 3 shows the nucleotide sequence of clones for the 22 kd zeins. Nucleotides are numbered starting with the first base of the initiator codon and are indicated on the right-hand margin. The sequences of cZ22A1 and cZ22B1 (previously named pZ22.1 and pZ22.3, respectively) were previously reported (Marks and Larkins, 1982). The complete nucleotide sequence of cZ22A1 is given, but only variable nucleotides at corresponding positions are listed for the other clones. Asterisks indicate positions where gaps were introduced in the sequence to maximize homology. The positions of the first nucleotides for cZ22B-1 and cZ22C-2 follow the colons. "An" indicates the position at which a sequence terminates in a poly(A) tail. Positions corresponding to the initiating methionine (Met) and the NH$_2$-terminus and -COOH terminus of the encoded polypeptide are indicated.

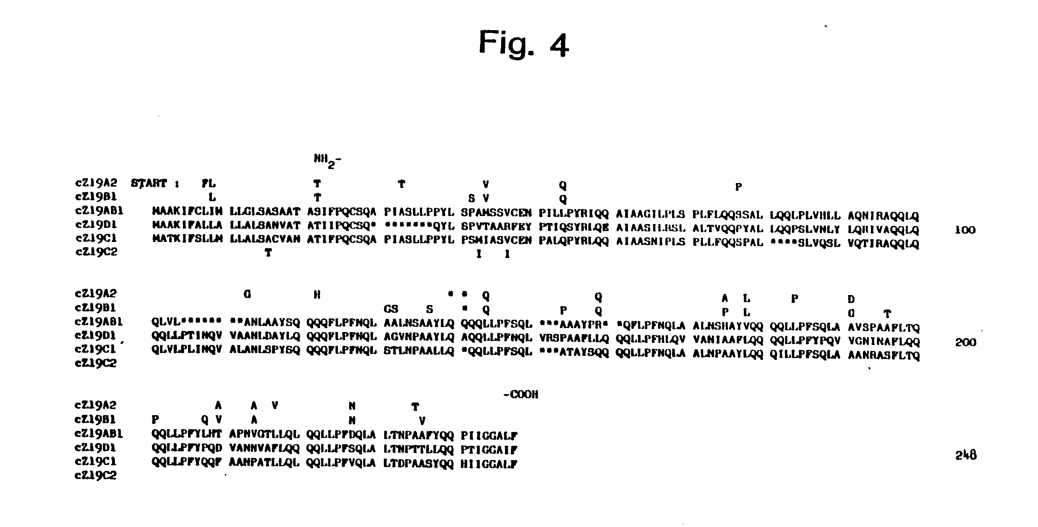

Figure 4 shows amino acid sequences deduced from the DNA sequence of clones for the Mr 19,000 zeins (Fig. 2). The complete amino acid sequences deduced from gZ19AB1, cZ19D1, and cZ19C1 are given. Only amino acid residues for cZ19A2 and cZ19B1 that differ from gZ19AB1 are shown as are only amino acid residues for cZ19C2 that differ from the sequence of cZ19C1. Asterisks indicate gaps introduced in the amino acid sequence to demonstrate homologies. The positions of the NH$_2$-terminus and -COOH terminus of the mature proteins are indicated.

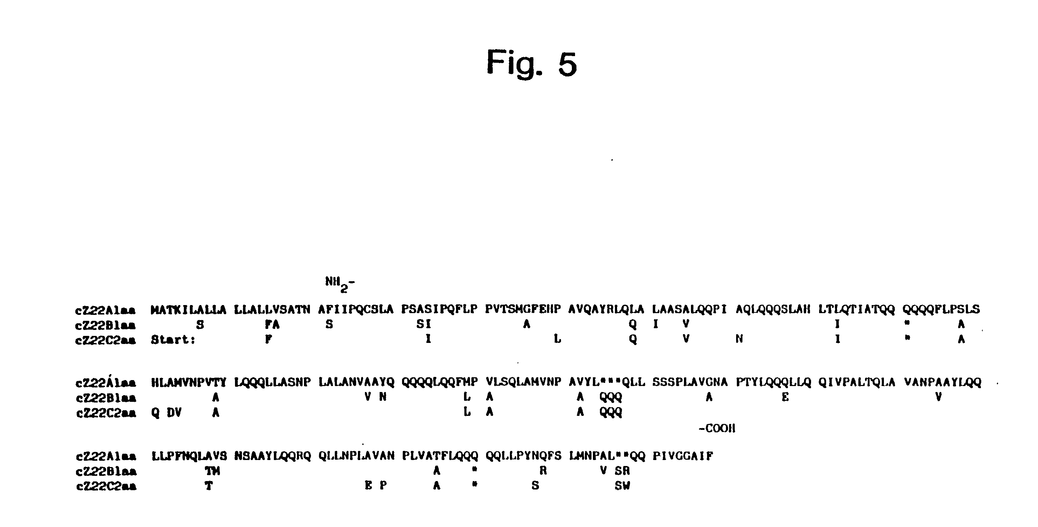

Figure 5 shows amino acid sequences deduced from DNA sequences of clones for the Mr 22,000 zeins (Fig. 3). The complete sequence of cZ22A1 is given in the standard single letter amino acid code, but only variable amino acid residues at corresponding positions are listed for the other sequences. The position corresponding to the NH$_2$-terminus of the mature protein is indicated.

## Example 2: Removal of homopolymer tails and cloning in M13

In order to generate efficiently translatable mRNA transcripts of a pre-zein gene or modified pre-zein gene it was discovered that the cloned sequences must be further tailored by removing the homopolymer tails generated during the cDNA cloning process. It was found to be sufficient to remove the homopolymer tail at the 5' end of the pre-zein coding sequence.

For this purpose the pUC8 vector containing the 19 pre-zein coding sequence was cut with EcoRI endonuclease. The EcoRI site of pUC8 lies within the same polylinker as the PstI site into which the pre-zein cDNA was originally cloned. Cleavage with EcoRI cuts the vector at the 5' end of the pre-zein gene. The EcoRI linearized plasmid was incubated with Bal31 exonuclease according to standard techniques for varying degrees of limited digestion. The digested linear plasmid was reisolated and cut with HindIII endonuclease. The HindIII site of the pUC8 polylinker lies on the 3' side of the pre-zein gene. The resulting

HindIII cleavage releases the pre-zein DNA coding insert in the form of a population of molecules having varying numbers of nucleotides removed from the 5' end.

The resulting population of 5'-deleted inserts was then cloned into M13mpl1, previously cut with SmaI and HindIII to generate a unidirectional cloning. Upon ligation of the inserts into SmaI/HindIII cut M13mp11, the sticky ends generated by HindIII cutting at the 3' end of the insert anneal with the HindIII cut end of the M13 vector while the blunt ends of the insert generated by Bal31 digestion can ligate by blunt end ligation to the SmaI cut end of the M13 vector.

Clones of individual inserts in M13mp11 were then sequenced at the 5' end of the insert to determine the amount of homopolymer tail removed by the Bal31 digestion. Three clones, representing three different degrees of digestion, were analyzed for ability to provide efficient translation, using the SP6 transcription system and frog oocyte translation system described infra in Example 4. The highest yield of zein was produced from transcripts derived from cDNA in which the entire 5' homopolymer tail had been removed as well as an additional two nucleotides of the 5' untranslated region of the pre-zein cDNA. Intermediate yields of zein were obtained using clones in which the homopolymer tails were partially removed. Therefore, removal of the homopolymer tail at the 5' end is of substantial importance for efficient translation.

## Example 3: Modification of pre-zein coding region by site-specific mutagenesis

M13 bacteriophage and reagents are available as a kit from Amersham Corp., Arlington Heights, Illinois, together with a handbook of instructions for carrying out various manipulations and isolations of single stranded and RF (double stranded) forms of M13 DNA. The handbook of instructions is hereinafter referred to as the "Amersham handbook". E. coli GM119 (dcm-6, dam-3, metB-1 thil, lac Y1 or lac 4Z, gal K2, gal T22, mt1-2, ton A2 or ton A31, tsx-1 or tsx-78, supE44, malR/F1KM) is a widely available E. coli strain used herein for the purpose of preparing non-methylated M13 DNA. The use of non-methylated DNA favors retention of the mutagenized strand during replication primed by a synthetic oligonucleotide as described, infra.

A culture of E. coli GM119 was grown from a single colony to an O.D. of 0.6 at 32° C in LB medium. Fifty $\mu$l of the O.D. 0.6 culture was added to 2 ml prewarmed LB medium and 5 $\mu$l of M13 phage suspension was added. The culture was grown overnight or for approximately 16 hours at 32° C. After the incubation, intact cells were removed by centrifugation. The supernatant contained phage from which single stranded M13 template DNA was prepared as described in the Amersham handbook.

Gapped heteroduplex DNA comprising unmethylated (+) (viral) strand and a methylated (-) strand was prepared by an annealing procedure. Minus (-) strand DNA was prepared from M13 RF DNA digested with HindIII and EcoRI endonucleases. The reaction mixture was extracted once with a Tris-EDTA buffer saturated with a chloroform/phenol mixture (equal parts by volume). The aqueous phase was then extracted twice with a 5-fold volume of diethyl ether, 0.2 volumes of 5 mM ammonium acetate and 2.5 volumes of ethanol were added to precipitate DNA during a 10-15 minute incubation in a dry ice-ethanol bath. The precipitate was collected by centrifugation, the supernatant was decanted and the tube containing the precipitate was inverted and allowed to air dry. The dried pellet was redissolved in 20 $\mu$l of Tris-EDTA buffer. The annealing reaction was initiated by combining 0.5 $\mu$g of (+) strand viral DNA (unmethylated) and 0.2 $\mu$g linearized RF DNA in 20 $\mu$l of 1x SSC. The mixture was overlayed with 50 $\mu$l of mineral oil and placed in a boiling water bath for 2 minutes. Immediately thereafter, the tube was transferred to a 65° bath and incubated for 4 hours or more to allow annealing to occur between the (+) and (-) DNA strands. Following annealing, the aqueous layer was transferred to a new tube and traces of mineral oil removed by two successive extractions with 5 volumes of diethyl ether. Residual ether was removed by applying mild vacuum conditions for 2 minutes. The DNA was precipitated by adding 50 $\mu$l cold ethanol to the reaction mixture and incubating the mixture for 20 minutes in dry ice-ethanol, collecting the precipitated DNA by centrifugation and air drying as before. The above procedure yields from 0.1 $\mu$g to 0.3 $\mu$g gapped heteroduplex DNA.

The mutagenic oligonucleotides were prepared according to standard techniques of DNA synthesis. Two mutagenic oligonucleotides were prepared, the first having the sequence GGTAGTTACTTTCGAAGT intended to provide an isoleucine to lysine change at amino acid position 44 of cZ19C1, and the second having the sequence GTAAATTTGTTAACCG, intended to encode the substitution of lysine for asparagine at amino acid position 167 in cZ19C1. The air dried heteroduplex mixture was dissolved in 1.5 $\mu$l of solution "A" [described by Zoller and Smith, Methods in Enzymology Vol. 100, p. 46B (1983)] and 2 $\mu$l containing approximately 15 pMole of mutagenic oligonucleotide was added, followed by 7.5 $\mu$l of DEPC treated water. (DEPC (Diethylpryocarbonate) added at about 0.01% (v/v) inactivates RNAse and hydrolyzes to yield traces of ethanol and $CO_2$.) The reaction was heated to 65° C and incubated at that temperature for 15 minutes.

The mixture was then annealed by transfer to room temperature for 20 minutes. To 10 $\mu$l of the mixture treated as described, the following were added: 1 $\mu$l each of 20 mM solutions of dATP, dCTP, dGTP and dTTP, 1.2 l of 10 mM ATP, 2.5 $\mu$l of 200 mM dTT (dithiothreitol), 1 $\mu$l DNA polymerase Klenow fragment and 1.2 $\mu$l of DEPC treated water. The mixture was incubated for 30 minutes at 22° C (room temperature). Following incubation 50 units of T4 DNA ligase was added and the mixture again incubated at 22° C (room temperature) for a period of 4 hours. The foregoing procedure provided closed circular DNA comprising ( + ) (viral) strands incorporating the mutated sequence derive from the mutagenic oligonucleotides.

The ligated heteroduplex mixture was used to transform E. coli JM103 cells, rendered competent for transformation according to the procedure described in the Amersham handbook. E. coli JM103 is widely available from a variety of sources. Two hundred $\mu$l of competent cells were mixed with 1 $\mu$l, 2 $\mu$l, 5 $\mu$l or 10 $\mu$l of heteroduplex mixture, incubated on ice for 40 minutes, then heat shocked by transfer to 42° C for 2 minutes, then returned to the ice bath. To each tube was then added 10 $\mu$l 100 mM IPTG and 50 mM 2% (w/v) X-gal, 100 $\mu$l of JM103 cells to provide a bacterial lawn, and 3 ml of 0.7% (w/v) fresh top agar, the latter kept melted at 50° C. The components of each tube were then quickly mixed and poured onto fresh petri plates containing M9 agar. After overnight incubation plaques were formed which appeared white, or colorless, for those phage containing an inserted zein gene.

The efficiency of the procedure yields from 4% to 10% of mutagenized sequences. Clones in which the mutated sequence have been incorporated were identified by the introduction of a new HindIII site in one case, or the loss of a pre-existing HindIII site in the other case, such that a rapid screening could be undertaken without the need for sequence determination of each clone. The restriction pattern of fragments produced by HindIII cleavage was compared after electrophoretic separation with the pattern of fragments produced by unmodified DNA. The resulting modified pre-zein coding sequences were removed from the M13 vector by an EcoRI-HindIII cut and cloned into the transcription vector SP6 (commercially available, e.g., from ProMega Biotech, Madison, Wisconsin) previously digested with EcoRI and HindIII endonucleases.

Example 4: Transcription and translation of modified pre-zein coding segment

The modified pre-zein coding segment, described in Example 3, was released from the M13 vector by digestion with EcoRI and HindIII endonucleases, and cloned into plasmid SP6, cut with EcoRI and HindIII. The transcription plasmid SP6 is commercially available from ProMega Biotec, Madison, Wisconsin. Other reagents and enzymes used for SP6 transcription are also available from ProMega Biotec. The modified pre-zein gene was transcribed from the SP6 promoter in a reaction containing the following: 40 ml Tris-HCl pH 7.5, 6 mM MgCl2, 2 mM spermadine, 10 mM dithiothreitol, 1 unit/ml RNAsin (trademark ProMega Biotec, Madison, Wisconsin) 100 $\mu$g/ml bovine serum albumin, 0.5 mM ATP, 0.5 mM TTP, 0.5 mM CTP, 0.1 mM GTP, 0.5 mM 7mGpppG and 15 units SP6 RNA polymerase. After 30 minutes incubation at 40° C, 5 additional units of SP6 polymerase and GTP to form a concentration of 0.5 mM were added. The reaction was continued for 1 hour at 40° C. The DNA template was removed by first adding RNAsin to the final concentration of 1 $\mu$g/$\mu$l and hen adding RNAse I to 20 $\mu$g/ml final concentration and incubating at 37° for 15 minutes. Under these conditions, approximately 0.2 $\mu$g of mRNA was transcribed per $\mu$g of plasmid DNA.

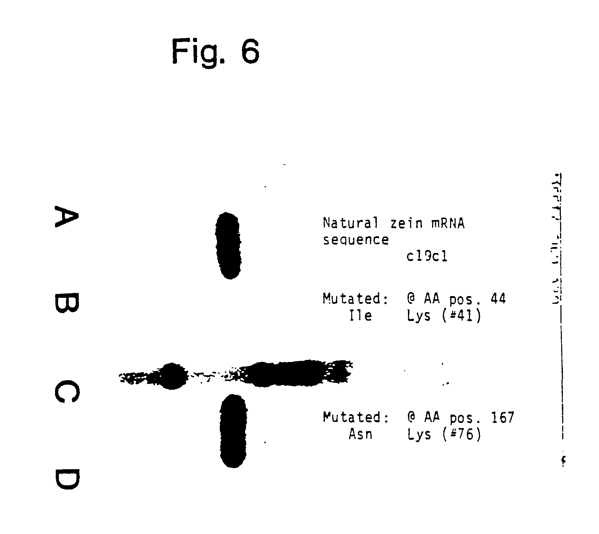

Translation of modified pre-zein mRNA was carried out in frog oocytes microinjected with mRNA as described by Hurkman et al. (1981). Translation was monitored by incorporation of tritiated leucine into protein after microinjection of mRNA. Membrane fractions were isolated from the oocytes and ethanol soluble proteins extracted and analyzed by electrophoresis on SDS acrylamide gels. The results are shown in Figure 6. Lane A shows that unmodified 19 kd zein mRNA was found as a single band corresponding to about 19 kd as judged by the migration of a series of markers of known molecular weight, shown in lane C. The result shows that unmodified zein was located within the membrane fraction of the oocytes and behaved as an ethanol soluble protein. Lane B showed the results with a modified pre-zein, having an isoleucine to lysine substitution at amino acid No. 44. No protein was found, indicating that the protein, if produced, did not have a structure allowing it to be translocated to endoplasmic reticulum. A control experiment in which the modified pre-zein mRNA encoding the same amino acid substitution was used in an in vitro translation system revealed that a protein of the proper length was obtained. Therefore, failure to detect product in the oocyte system was not due to a defect in the modified zein mRNA, since the latter was translated normally in an in vitro system. In lane D, oocytes were injected with a modified pre-zein mRNA in which an asparagine residue was replaced by a lysine at amino acid position 159. The result indicates normal translation translocation and physical properties similar to unmodified zein by all the criteria measured by the experiment.

The sequence and postulated folded configuration of modified and unmodified Z19C1 zein are shown in Fig. 7. The amino acid abbreviations are shown in Table 2. Cross-bars show hydrogen bonds between residues on adjacent domains of α-helix, postulated to stabilize the highly compact central position having repeated segments of amino acid sequence. Asterisks show the positions where lysine was substituted, as described in the foregoing examples. The compactness of the tertiary structure can be more fully appreciated by rolling the figure into a cylinder around the long axis of the page.

While the invention has been exemplified by a modified 19 kd zein containing lysine, other zeins within the group of 19 kd and 22 kd zeins may be modified, lysine may be introduced by other means, at other locations, by additions and insertions as well as by substitutions within the amino acid sequence, all according to the teachings herein and expedients known to those of ordinary skill in the art mutatis mutandis. Such modifications and variations leading to a modified 19 kd or 22 kd zein comprising a lysine residue are deemed to fall within the scope of the invention, as set forth in the claims.

TABLE 2

| AMINO ACID ABBREVIATIONS | |
|---|---|
| A = Ala = Alanine | M = Met = Methionine |
| C = Cys = Cysteine | N = Asn = Asparagine |
| D = Asp = Aspartic Acid | P = Pro = Proline |
| E = Glu = Glutamic Acid | Q = Gln = Glutamine |
| F = Phe = Phenylalanine | R = Arg = Arginine |
| G = Gly = Glycine | S = Ser = Serine |
| H = His = Histidine | T = Thr = Threonine |
| I = Ile = Isoleucine | V = Val = Valine |
| K = Lys = Lysine | W = Try = Tryptophan |
| L = Leu = Leucine | Y = Tyr = Tyrosine |

**Claims**

1. A modified 19kd or 22kd zein, the amino acid composition of which comprises lysine and which has a structure permitting the modified zein to be translocated to the endoplasmic reticulum in a host cell.

2. A modified zein according to claim 1 wherein said lysine is located within an internal repeated region of the zein.

3. A modified zein according to claim 1 wherein said lysine is located in a COOH-terminal non-repeated region of the zein.

4. A modified zein according to claim 1 wherein said lysine is located in an NH₂-terminal non-repeated region of the zein.

5. A modified zein according to any of claims 1 to 4 in the endoplasmic reticulum of a cell or tissue.

6. A modified zein according to claim 1 having an amino acid sequence containing lysine as follows:

```
   NH2-T  I  F  P  Q  C  S  Q  A  P  I  A  S  L  L  P  P  Y
      L  P  S  M  I  A  S  V  C  E  N  P  A  L  Q  P  Y  R  L  Q
      Q  A  I  A  A  S  N  I  P  L  S  P  L  L  F  Q  Q  S  P  A
      L  S  L  V  Q  S  L  V  Q  T  I  R  A  Q  Q  L  Q  Q  L  V
      L  P  L  I  N  Q  V  A  L  A  N  L  S  P  Y  S  Q  Q  Q  Q
      F  L  P  F  N  Q  L  S  T  L  N  P  A  A  L  L  Q  Q  Q  L
      L  P  F  S  Q  L  A  T  A  Y  S  Q  Q  Q  Q  L  L  P  F  K
      Q  L  A  A  L  N  P  A  A  Y  L  Q  Q  Q  I  L  L  P  F  S
      Q  L  A  A  A  N  R  A  S  F  L  T  Q  Q  Q  L  L  P  F  Y
      Q  Q  F  A  A  N  P  A  T  L  L  Q  L  Q  Q  L  L  P  F  V
      Q  L  A  L  T  D  P  A  A  S  Y  Q  Q  H  I  I  G  G  A  L
      F-COOH.
```

7.  A DNA segment encoding a 19kd or 22kd pre-zein wherein the pre-zein coding sequence comprises a codon for lysine, the transcription and translation of said coding sequence in a host cell yielding a zein located within a protein body of the endoplasmic reticulum of said host cell.

8.  The DNA segment of claim 7 wherein said codon for lysine is located within the DNA sequence which encodes an internal repeated region of said pre-zein.

9.  The DNA segment of claim 7 wherein said codon for lysine is located within the DNA sequence which encodes the -COOH terminus of the pre-zein.

10. The DNA segment of claim 7 wherein said codon for lysine is located within the DNA sequence which encodes the NH$_2$-terminus of the pre-zein.

11. The DNA segment of any of claims 7 to 10 which is contained in a host cell.

12. The DNA segment of any of the claims 7 to 11 wherein said codon for lysine is substituted for a codon for a polar or moderately non-polar amino acid.

13. The DNA segment of claim 12 wherein said codon for lysine is substituted for a codon for asparagine.

14. The DNA segment according to claim 7 encoding the 19kd pre-zein of cZ19C1 modified to contain a lysine codon substituted for an asparagine codon at amino acid codon position 159 wherein said DNA segment encodes the amino acid sequence containing lysine as follows:-

```
   NH2-T  I  F  P  Q  C  S  Q  A  P  I  A  S  L  L  P  P  Y
      L  P  S  M  I  A  S  V  C  E  N  P  A  L  Q  P  Y  R  L  Q
      Q  A  I  A  A  S  N  I  P  L  S  P  L  L  F  Q  Q  S  P  A
      L  S  L  V  Q  S  L  V  Q  T  I  R  A  Q  Q  L  Q  Q  L  V
      L  P  L  I  N  Q  V  A  L  A  N  L  S  P  Y  S  Q  Q  Q  Q
      F  L  P  F  N  Q  L  S  T  L  N  P  A  A  L  L  Q  Q  Q  L
      L  P  F  S  Q  L  A  T  A  Y  S  Q  Q  Q  Q  L  L  P  F  K
      Q  L  A  A  L  N  P  A  A  Y  L  Q  Q  Q  I  L  L  P  F  S
      Q  L  A  A  A  N  R  A  S  F  L  T  Q  Q  Q  L  L  P  F  Y
      Q  Q  F  A  A  N  P  A  T  L  L  Q  L  Q  Q  L  L  P  F  V
      Q  L  A  L  T  D  P  A  A  S  Y  Q  Q  H  I  I  G  G  A  L
      F-COOH.
```

**Revendications**

1.  Zéine de 19kd ou 22kd modifiée, dont la composition en acides aminés comprend la lysine et qui a une structure permettant à la zéine modifiée de subir une translocation dans le réticulum endoplasmique d'une cellule hôte.

2. Zéine modifiée selon la revendication 1, dans laquelle ladite lysine est située dans une région récurrente interne de la zéine.

3. Zéine modifiée selon la revendication 1, dans laquelle ladite lysine est située dans une région non récurrente COOH-terminale de la zéine.

4. Zéine modifiée selon la revendication 1, dans laquelle ladite lysine est située dans une région non récurrente NH2-terminale de la zéine.

5. Zéine modifiée selon l'une quelconque des revendications 1 à 4, dans le réticulum endoplasmique d'une cellule ou d'un tissu.

6. Zéine modifiée selon la revendication 1, possédant une séquence d'acides aminés contenant la lysine, qui est la suivante :

```
   NH2-T   I   F   P   Q   C   S   Q   A   P   I   A   S   L   L   P   P   Y
 L   P   S   M   I   A   S   V   C   E   N   P   A   L   Q   P   Y   R   L   Q
 Q   A   I   A   A   S   N   I   P   L   S   P   L   L   F   Q   Q   S   P   A
 L   S   L   V   Q   S   L   V   Q   T   I   R   A   Q   Q   L   Q   Q   L   V
 L   P   L   I   N   C   V   A   L   A   N   L   S   P   Y   S   Q   Q   Q   Q
 F   L   P   F   N   Q   L   S   T   L   N   P   A   A   L   L   Q   Q   Q   L
 L   P   F   S   Q   L   A   T   A   Y   S   Q   Q   Q   Q   L   L   P   F   K
 Q   L   A   A   L   N   P   A   A   Y   L   Q   Q   Q   I   L   L   P   F   S
 Q   L   A   A   A   N   R   A   S   F   L   T   Q   Q   Q   L   L   P   F   Y
 Q   Q   F   A   A   N   P   A   T   L   L   Q   L   Q   Q   L   L   P   F   V
 Q   L   A   L   T   D   P   A   A   S   Y   Q   Q   H   I   I   G   G   A   L
 F-COOH.
```

7. Segment d'ADN codant pour une pré-zéine de 19 kd ou 22 kd, dans lequel la séquence codant pour la pré-zéine comprend un codon pour la lysine, la transcription et la traduction de ladite séquence codante dans une cellule hôte donnant une zéine située dans un corps protéique du réticulum endoplasmique de ladite cellule hôte.

8. Segment d'ADN selon la revendication 7, dans lequel ledit codon pour la lysine est situé dans la séquence d'ADN qui code pour une région récurrente interne de ladite pré-zéine.

9. Segment d'ADN selon la revendication 7, dans lequel ledit codon pour la lysine est situé dans la séquence d'ADN qui code pour l'extrémité -COOH de la pré-zéine.

10. Segment d'ADN selon la revendication 7, dans lequel ledit codon pour la lysine est situé dans la séquence d'ADN qui code pour l'extrémité NH2- de la pré-zéine.

11. Segment d'ADN selon l'une quelconque des revendications 7 à 10, qui est contenu dans une cellule hôte.

12. Segment d'ADN selon l'une quelconque des revendications 7 à 11, dans lequel on substitue ledit codon pour la lysine à un codon pour un acide aminé polaire ou modérément non polaire.

13. Segment d'ADN selon la revendication 12, dans lequel on substitue ledit codon pour la lysine à un codon pour l'aspargine.

14. Segment d'ADN selon la revendication 7, codant pour la pré-zéine de 19 kd de cZ19C1 modifiée pour contenir un codon de lysine substitué à un codon d'asparagine en position 159 du codon d'acides aminés, dans lequel ledit segment d'ADN code pour la séquence d'acides aminés contenant la lysine, qui est la suivante :

```
NH2-T I F P Q C S Q A P I A S L L P P Y
L  P  S M I A S V C E N P A L Q P Y R L Q
Q  A  I A A S N I P L S P L L F Q Q S P A
L  S  L V Q S L V Q T I R A Q Q L Q Q L V
L  P  L I N Q V A L A N L S P Y S Q Q Q Q
F  L  P F N Q L S T L N P A A L L Q Q Q L
L  P  F S Q L A T A Y S Q Q Q Q L L P F K
Q  L  A A L N P A A Y L Q Q Q I L L P F S
Q  L  A A A N R A S F L T Q Q Q L L P F Y
Q  Q  F A A N P A T L L Q L Q Q L L P F V
Q  L  A L T D P A A S Y Q Q H I I G G A L
F-COOH.
```

## Patentansprüche

1. Modifiziertes Zein mit 19 kd oder 22 kd, in dessen Aminosäurezusammensetzung Lysin enthalten ist und das eine Struktur aufweist, die es ermöglicht, daß das modifizierte Zein in das endoplasmatische Retikulum in einer Wirtszelle übertragen werden kann.

2. Modifiziertes Zein nach Anspruch 1, in welchem das Lysin innerhalb einer internen wiederholten Region des Zeins angeordnet ist.

3. Modifiziertes Zein nach Anspruch 1, in welchem das Lysin in einer COOH-terminalen, nicht-wiederholten Region des Zeins angeordnet ist.

4. Modifiziertes Zein nach Anspruch 1, in welchem das Lysin in einer NH$_2$-terminalen, nicht-wiederholten Region des Zeins angeordnet ist.

5. Modifiziertes Zein nach einem der Ansprüche 1 bis 4 in dem endoplasmatischen Retikulum einer Zelle oder eines Gewebes.

6. Modifiziertes Zein nach Anspruch 1 mit der folgenden, Lysin enthaltenden Aminosäuresequenz:

```
NH2-T I F P Q C S Q A P I A S L L P P Y
L  P  S M I A S V C E N P A L Q P Y R L Q
Q  A  I A A S N I P L S P L L F Q Q S P A
L  S  L V Q S L V Q T I R A Q Q L Q Q L V
L  P  L I N Q V A L A N L S P Y S Q Q Q Q
F  L  P F N Q L S T L N P A A L L Q Q Q L
L  P  F S Q L A T A Y S Q Q Q Q L L P F K
Q  L  A A L N P A A Y L Q Q Q I L L P F S
Q  L  A A A N R A S F L T Q Q Q L L P F Y
Q  Q  F A A N P A T L L Q L Q Q L L P F V
Q  L  A L T D P A A S Y Q Q H I I G G A L
F-COOH.
```

7. DNA-Segment, das für ein Pre-Zein mit 19 kd oder 22 kd codiert, in welchem die für das Pre-Zein codierende Sequenz ein Codon für Lysin enthält, wobei die Transkription und Translation dieser Codierungssequenz in einer Wirtszelle ein Zein ergibt, das innerhalb eines Proteinkörpers des endoplasmatischen Retikulums dieser Wirtszelle lokalisiert ist.

8. DNA-Segment nach Anspruch 7, bei welcher dieses Codon für Lysin innerhalb der DNA-Sequenz lokalisiert ist, die für eine interne wiederholte Region dieses Pre-Zeins codiert.

9. DNA-Segment nach Anspruch 7, bei welcher dieses Codon für Lysin innerhalb der DNA-Sequenz lokalisiert ist, die für das -COOH-Ende des Pre-Zeins codiert.

10. DNA-Segment nach Anspruch 7, bei welchem dieses Codon für Lysin innerhalb der DNA-Sequenz lokalisiert ist, die für das NH₂-Ende des Pre-Zeins codiert.

11. DNA-Segment nach einem der Ansprüche 7 bis 10, das in einer Wirtszelle enthalten ist.

12. DNA-Segment nach einem der Ansprüche 7 bis 11, bei welchem dieses Codon für Lysin ein Codon für eine polare oder mäßig unpolare Aminosäure ersetzt.

13. DNA-Segment nach Anspruch 12, bei welchem dieses Codon für Lysin ein Codon für Asparagin ersetzt.

14. DNA-Segment nach Anspruch 7, das für das 19 kd Pre-Zein von cZ19C1 codiert, das so modifiziert ist, daß es ein Lysincodon anstelle eines Asparagincodons an der Aminosäure-Codonstelle 159 enthält, wobei das DNA-Segment für die folgende, Lysin enthaltende Aminosäuresequenz codiert:

```
        NH2-T   I   F   P   Q   C   S   Q   A   P   I   A   S   L   L   P   P   Y
      L   P   S   M   I   A   S   V   C   E   N   P   A   L   Q   P   Y   R   L   Q
      Q   A   I   A   A   S   N   I   P   L   S   P   L   L   F   Q   Q   S   P   A
      L   S   L   V   Q   S   L   V   Q   T   I   R   A   Q   Q   L   Q   Q   L   V
      L   P   L   I   N   Q   V   A   L   A   N   L   S   P   Y   S   Q   Q   Q   Q
      F   L   P   F   N   Q   L   S   T   L   N   P   A   A   L   L   Q   Q   Q   L
      L   P   F   S   Q   L   A   T   A   Y   S   Q   Q   Q   Q   L   L   P   F   K
      Q   L   A   A   L   N   P   A   A   Y   L   Q   Q   Q   I   L   L   P   F   S
      Q   L   A   A   A   N   R   A   S   F   L   T   Q   Q   Q   L   L   P   F   Y
      Q   Q   F   A   A   N   P   A   T   L   L   Q   L   Q   Q   L   L   P   F   V
      Q   L   A   L   T   D   P   A   A   S   Y   Q   Q   H   I   I   G   G   A   L
      F-COOH.
```

Figure 1

Consensus Amino Acid Sequence of Zein Proteins

| Zein protein | Signal Sequence | Sequence positions |
|---|---|---|
| Z22 | M A T K I L S L L A L L A L F A S A T N A | 1-21 |
| Z19 | M A A K I F C L I M L L G L S A S A A T A | 1-21 |

N-Terminal Turn

| | | |
|---|---|---|
| Z22 | S I I P Q C S L A P . S S I I P Q F L P P V T S M A F E H P A V Q A Y R | 22-55 |
| Z19 | S I F P Q C S Q A P I A S L L P P Y . S P A M S S V C E N P I L L P Y R | 22-57 |

Repeat Sequences

| | | | |
|---|---|---|---|
| Z22 and Z19 | | F A A A | 57-237 |
| | Q Q | L P N Q L A N S P A Y L Q Q | |
| 9 repeats | | L F L V | 58-225 |

C-Terminal Turn

| | | |
|---|---|---|
| Z22 | Q Q Q L L P Y N R F S L M N P V L S R Q Q P I V G G A I F | 238-266 |
| Z29 | . . . . . . . . . . . . . . . . . . . . . . Q Q P I I G G A L F | 226-235 |

# Fig. 2-1

```
                                                            Met

        -65                                              -1+1
cZ19A2           .           .          .        .        .        .    START :              T   C
cZ19B1   START:                          C                  G        C                        C
gZ19AB1        ATCCCACATATTATTGAGACCAACTACCAATATAGAAAGCA****CAATATTGTACCAATA ATG CCA GCC AAA ATA TTT TGC CTC ATT ATG CTC CTT GCT
cZ19D1                       ACATAGTGAACTACACTAGCAACATCTTAGCACCA ATG GCA GCC AAG ATT TTT GCC CTC CTT GCC CTC CTT GCT           + 39
cZ19C2                    ACAAGCAACATAGAAAGTGGAATCCAGTAGCAACAATAGAGCCAACA ATG GCG ACC AAG ATA TTT TGC CTC CTT ATG CTC CTT GCT
cZ19C1                START:                            C

                            NH2-
cZ19A2                    C     C                                A                              G           T
cZ19B1                          C                    G                                          G           A
gZ19AB1  CTT TCT CCA AGT GCT GCT ACG GCG AGC ATT TTC CCG CAA TGC TCA CAA GCT CCT ATA GCT TCC CTT CTT CCC CCA TAC CTC TCA CCA CCG
cZ19D1   CTT TCA GCA AAC GTT GCT ACC GCG ACT ATT ATT CCA CAA TGC TCA CAA *** *** *** *** *** *** *** *** CAA TAC CTC TCT CCA CCG   +129
cZ19C2   CTT TCT ACA TGT GTT GCT AAC GCG ACA ATT TTC CCT CAA TGC TCA CAA GCT CCT ATA GCT TCC CTT CTT CCC CCA TAC CTT CCA TCA ATT
cZ19C1        G                                                                                                              G

cZ19A2   G           G           C           A
cZ19B1   G       G               C       C   A                                       T
gZ19AB1  ATG TCT TCA CTA TGT GAA AAT CCA ATT CTT CTA CCC TAC ACG ATC CAA CAG GCA ATC GCA GCA CGC ATC TTA CCT TTA TCA CCC TTG TTC
cZ19D1   ACA GCC GCG AGA TTT GAA TAC CCA ACT ATA CAA TCC TAC AGG CTA CAA CAG GCC ATC GCA GCA AGC ATC TTA CGG TCG TTA GCA TTG ACT   +219
cZ19C2   ATA GCT TCA ATA TGT GAA AAC CCA CCT CTT CAA CCA TAT ACG CTT CAA CAA GCA ATC GCA GCA AGC AAC ATA CCT TTA TCG CCC TTG TTC
cZ19C1        G                               G   C

cZ19A2         C  G                                                       T                                    T
cZ19B1                                                        A                                                T
gZ19AB1  CTC CAA CAA TCA TCA GCC CTA TTA CAA CAG TTA CCT TTG GTC CAT TTG TTG GCA CAA AAC ATC AGG GCA CAA CAA CTA CAA CAA CTC GTC
cZ19D1   GTC CAA CAA CCA TAT GCC CTA TTG CAA CAA CCA TCC TTA GTC AAT CTA TAT CTC CAA AGA ATC GTA GCA CAA CAA CTA CAA CAA CAA TTG   +309
cZ19C2   TTT CAA CAA TCG CCA GCC CTA *** *** *** *** TCT TTG GTG CAG TCA TTG GTA CAA ACC ATC AGG GCA CAA CAA CAG GTG CAG CAA CTC GTG
cZ19C1
```

EP 0 208 418 B1

# Fig.2-2

```
cZ19A2                                                      G              A            T          T                 G
cZ19B1                                                                                  C          T                 G   T
aZ19AB1   CTA *** *** *** *** *** *** *** *** GCA AAC CTT GCT GCC TAC TCT CAG CAA CAA CAG TTT CTG CCA TTC AAC CAA CTA GCT GCA TTG
cZ19D1    CTT CCA ACA ATC AAT CAA GTA GTT GCA GCG AAC CTT GAT GCT TAC CTC CAG CAA CAA CAA TTT CTT CCA TTC AAT CAA CTA GCT GCG GTG   .399
cZ19C2    CTA CCT CTG ATC AAC CAA GTA GCT CTG GCA AAC CTT TCT CCC TAC TCT CAG CAA CAA CAA TTT CTT CCA TTC AAC CAA CTG TCT ACA CTG
cZ19C1


cZ19A2                                *** ***           A                                                    A
cZ19B1                    T           A ***             A                                          C         A
aZ19AB1   AAC TCT GCT GCT TAT TTG CAG CAA CAA CAA CTA CTA CCA TTC AGC CAG CTA *** *** *** GCT GCT GCC TAC CCC CCG *** *** CAA TTT
cZ19D1    AAC CCT CCT GCT TAC TTG CAG GCA CAA CAG CTA CTA CCA TTC AAC CAA CTT CTC AGG AGC CCT GCT GCC TTC TTA CTG CAG CAA CAC TTC   .489
cZ19C2    AAC CCT GCT GCT TAT TTG CAG *** CAA CAA CTA TTA CCA TTT AGC CAG CTA *** *** *** GCT ACT GCC TAC TCT CAG CAA CAA CAA CTT
cZ19C1


cZ19A2                 T                              GC        T        G       G       C                                 A
cZ19B1                                               C         T        G                                                 G
aZ19AB1   CTT CCA TTC AAC CAA CTG GCA GCA TTG AAC TCT CAT GCT TAT GTA CAA CAA CAA CAA CTA CTA CCA TTC AGC CAG CTA CCT GCT GTG ACC
cZ19D1    TTG CCA TTC CAT CTA CAA GTT CTG CCA AAC ATT GCT GCT TTC TTG CAA CAA CAA CAA TTG CTG CCA TTT TAC CCA CAG GTT CTG GGA AAC   .579
cZ19C2    CTT CCA TTT AAC CAA TTG GCC CCA CTG AAC CCC CCT GCT TAT TTG CAG CAG CAA ATA CTA CTA CCA TTT AGC CAG CTA CCT CCA GCA AAC
cZ19C1


cZ19A2                                            A            G                C       G
cZ19B1              A           A   C             A            GT               C
aZ19AB1   CCT GCT GCC TTC TTG ACA CAG CAA CAG TTG TTG CCG TTC TAC CTC CAC ACT GCG CCT AAC GTT GGC ACC CTC TTA CAA CTG CAA CAA TTG
cZ19D1    ATT AAC GCC TTC TTG CAA CAG CAA CAG TTG CTG CCA TTC TAC CCA CAG GAT GTG GCA AAC AAT GTC GCC TTC TTA CAA CAA CAA CAA TTG   .669
cZ19C2    CGT GCT TCC TTC TTG ACA CAG CAA CAG TTG CTG CCT TTC TAC CAG CAG TTT GCG GCT AAC CCC GCA ACC CTC TTA CAA CTA CAA CAA TTG
cZ19C1
```

# Fig. 2-3

-COOH

```
cZ19A2                                                              A
cZ19B1                    A                              T
gZ19AB1   CTG CCA TTC GAC CAA CTT GCT TTG ACA AAC CCA GCA GCG TTC TAC CAA CAA CCC ATC ATT GGT GGT GCC CTC TTT TAG
cZ19D1    CTG CCA TTT AGC CAA CTT GCT TTG ACG AAT CCT ACC ACC TTA TTG CAG CAG CCC ACC ATT GGT GGT GCC ATC TTC TAG    +747
cZ19C2    TTG CCC TTT GTC CAA CTT GCT TTG ACA GAC CCA GCG GCC TCC TAC CAA CAA CAC ATC ATT GGT GGT GCC CTC TTT TAG
cZ19C1
```

```
cZ19A2                        G       G A    G                                     Anend
cZ19B1        T                        AT    G                                       Anend
gZ19AB1   ATTGCTTATCAGTTATAGTTCAATAATAAAGTTTTTTTTGCTGATATTTGTGCCTTCCCAGAAATAAGAAAGTACATTTCTAGATTCTTATGTGCTTCTACTCTCCAA...    +854
cZ19D1    ATTTTTTATCCTTTATACTCTAATAATAAAGTTCTCATACTGATATGTGCCAACTTCTCAGTAATAAAACATTAGACATCTATATTTTATTAtend
cZ19C2    ATTGCTTATTAGTTGTAATTCAATAATAAAGTTTTTTTGCATGATGTATGTGCCCAACCAGAAATAAGAAGTTACATTTCCAntend
cZ19C1                                                            AGATTCTAATGTGATATTGGT
```

# Fig. 3

EP 0 208 418 B1

Met

-67                                                                                     -1+1

cI22A1  TCTCCCATAATATTTTCAOCATTCAAAAACACACCAAOCOAAOCOCACTAOCAACOACCTAACACCA ATO OCT ACC AAO ATA TTA GCC CTC CTT OCO CTT CTT GCC
cI22B1                                                                    start: A         A                          T                                    O    + 39
cI22C2                                                                                      start:

NH₂-

cI22A1  CTT TTA OTO AOC OCA ACA AAT OCO TTC ATT ATT CCA CAO TOC TCA CTT OCT CCT AOT OCC AOT ATT CCA CAO TTC CTC CCA CCA OTT ACT
cI22B1      T  C                     C                A                          T   T                                                  +129
cI22C2      T                        C                A                          T   A

cI22A1  TCA ATO OOT TTC OAA CAT CCA OCO OTO CAA OCC TAC AOO CTA CAA CTA OCO CTT OCO OCO AOC OCC TTA CAA CAA CCA ATT OCC CAA TTO
cI22B1      CC          C   T         T                A   A            T                                                      +219
cI22C2      C           C  T  T                        A       A        A   T                              AA

cI22A1  CAA CAA CAA TCC TTO OCA CAT CTA ACC CTA CAA ACC ATT OCA ACO CAA CAA CAA CAA CAA CAO TTT CTO CCA TCA CTO AOC CAC CTA OCC
cI22B1                          A A        C                O ***           C   A       O                                        +309
cI22C2                          A          C                O ***           C   A       O           A         AT

cI22A1  ATO OTO AAC CCT OTC ACC TAC TTO CAA CAO CAO CTO CTT OCA TCC AAC CCA CTT OCT CTO OCO AAC OTA OCT OCA TAC CAO CAA CAA CAA
cI22B1                O                                              A   A       T   A                          +399
cI22C2  O             O                                              A               A

cI22A1  CAO CTO CAA CAO TTT ATO CCA OTO CTC AOT CAA CTA OCC ATO OTO AAC CCT OCC OTC TAC CTA *** *** *** CAA CTA CTT TCA TCT AOC
cI22B1  A                 C       C   T                            C       CAA CAO CAA        O          +489
cI22C2  A T   O           C       C                                C       CAA CAO CAA        O

cI22A1  CCO CTC OCO OTO OOC AAT OCA CCT ACO TAC CTA CAA CAA CAO TTO CTO CAA CAA ATT OTA CCA OCT CTO ACT CAO CTA OCT OTO OCA AAC
cI22B1          T   C           A   O       O A   T       O                A                       +579
cI22C2  T       T   T           A   O       A           O

cI22A1  CCT OCT OCC TAC TTA CAA CAO TTO CTT CCA TTC AAC CAA CTO OCT OTO TCA AAC TCT OCT OCO TAC CTA CAA CAO COA CAA CAO TTA CTT
cI22B1      T           O      C               A A   A   O       T                              +669
cI22C2                  O      C                       A       O

cI22A1  AAT CCA TTO OCA OTO OCT AAC CCA TTO OTC OCT ACC TTC CTO CAO CAO CAA CAA CAA TTO CTO CCA TAC AAC CAO TTC TCT TTO ATO AAC
cI22B1                              O       A       ***               O                    T    +759
cI22C2      C A  A       C A                OO      A       ***                A       O                       T

-COOH

cI22A1  CCT OCC TTO *** *** CAO CAA CCC ATC OTT OOA OOT OCC ATC TTT TAO ATTACATATOAOATOTACTCOACAATOOTOCCCTCATA :end
cI22B1      T     TCO AOO                                          T          CCOOCATOTOTTTCCTA    +862
cI22C2            TCO TOO                                          T          CCOOCATOTOTTTCCTA

cI22B1  OAAATAATCAATATATTOOTOAn
cI22C2  OAAATAAACAATATATTOATTOAOATTTATCTCOATATATTOTTOAACTATOTTCATCATATAAATAATTOAAAACATCAAATCOTAATTATAAACTAn    +960

# Fig. 4

```
                                     NH2-
cZ19A2   START :   FL              T              T              V              Q                              P
cZ19B1             L              T                            S V             Q
cZ19AB1  MAAKIFCLIM LLGLSASAAT ASIFPQCSQA PIASLLPPYL SPAMSSVCEN PILLPYRIQQ AIAAGILPLS PLFLQQSSAL LQQLPLVHLL AQNIRAQQLQ
cZ19D1   MAAKIFALLA LLALSANVAT ATIIPQCSQ* *******QYL SPVTAARFKY PTIQSYRLQE AIAASILISL ALTVQQPYAL LQQPSLVNLY LQHIVAQQLQ    100
cZ19C1   MATKIFSLLM LLALSACVAN ATIFPQCSQA PIASLLPPYL PSMIASVCEN PALQPYRLQQ AIAASNIPLS PLLFQQSPAL ****SLVQSL VQTIRAQQLQ
cZ19C2                        T                                      I  I


cZ19A2                    G          H                     *  *  Q                Q               A  L      P          D
cZ19B1                                          GS      S      *  Q          P    Q               P  L         QQLLPFSQLA  Q      T
cZ19AB1  QLVL****** **ANLAAYSQ QQQFLPFNQL AALNSAAYLQ QQQLLPFSQL ***AAAYPR* *QFLPFNQLA ALNSHAYVQQ QQLLPFSQLA AVSPAAFLTQ
cZ19D1   QQLLPTINQV VAANLDAYLQ QQQFLPFNQL AGVNPAAYLQ AQQLLPFNQL VRSPAAFLLQ QQLLPFHLQV VANIAAFLQQ QQLLPFYPQV VGNINAFLQQ    200
cZ19C1   QLVLPLINQV ALANLSPYSQ QQQFLPFNQL STLNPAALLQ *QQLLPFSQL ***ATAYSQQ QQLLPFNQLA ALNPAAYLQQ QILLPFSQLA AANRASFLTQ
cZ19C2

                                                            -COOH
cZ19A2               A     A  V              N          T
cZ19B1     P      Q  V     A                 N          V
cZ19AB1  QQLLPFYLHT APNVGTLLQL QQLLPFDQLA LTNPAAFYQQ PIIGGALF
cZ19D1   QQLLPFYPQD VANHVAFLQQ QQLLPFSQLA LTNPTTLLQQ PTIGGAIF
cZ19C1   QQLLPFYQQF AANPATLLQL QQLLPFVQLA LTDPAASYQQ HIIGGALF                                                            248
cZ19C2
```

EP 0 208 418 B1

# Fig. 5

$NH_2^-$

```
cZ22Alaa  MATKILALLA LLALLVSATN AFIIPQCSLA PSASIPQFLP PVTSMGPEHP AVQAYRLQLA LAASALQQPI AQLQQQSLAH LTLQTIATQQ QQQQFLPSLS
cZ22Blaa       S          FA  S              SI              A              Q I  V                        I         *      A
cZ22C2aa  Start:          F                  I                     L        Q    V    N                   I         *      A


cZ22Alaa  HLAMVNPVTY LQQQLLASNP LALANVAAYQ QQQQLQQFMP VLSQLAMVNP AVYL***QLL SSSPLAVGNA PTYLQQQLLQ QIVPALTQLA VANPAAYLQQ
cZ22Blaa       A                     V N          L  A          A  QQQ            A          E                        V
cZ22C2aa  Q DV      A                          L  A          A  QQQ
                                                                              -COOH

cZ22Alaa  LLPFNQLAVS NSAAYLQQRQ QLLNPLAVAN PLVATFLQQQ QQLLPYNQFS LMNPAL**QQ PIVGGAIF
cZ22Blaa       TM                              A     *          R        V SR
cZ22C2aa       T                     E P        A     *          S        SW
```

# Fig. 6

A

B

C

D

Natural zein mRNA
sequence
        c19c1

Mutated:  @ AA pos. 44
    Ile    Lys (#41)

Mutated:  @ AA pos. 167
    Asn    Lys (#76)

# Fig. 7

19cl (MATURE)

```
                                                                    F(COOH)
                                                                    L
                                                                    A
                          Q Q                                       G
                       S    Q                                       G
             Q         Y — Q                                        I
    Q     S    P    F         Q  Q L                                I
    L     P    S    L    Q       P        T    Q Q                  H
    L     A    L    P    Q       F        L      Q                  Q
    P     L    F    F    Q       F   N*K  F      L                  Q
    S     S — N    N    S — N°K  Q — S    L      P                  Y
    L     L    A    N — Y   Q — S    A    F      L                  S
    P     V    L    Q   A   L    R    Y — P                         A
    I     Q    A    L   T   A    N — Q                              A
    N —   S    V    S   A   A    A    Q                             P
    S .   V    Q — T   L   L    A    F                              D
    A     Q — N    L   Q — N    A    A                              T
    A     T    I    N — S   P    A    A                             L
    I     I    L    P   F   A    L    A                             A
    A     R    P    A.  P   A    Q — N                              L
    Q     A    L    A   L   Y — S    P                              Q
    Q' —  Q    V    L   L·  L    F    A                             V
    L     Q    L    Q   Q   Q    P    T —                           F
    R     L      Q    Q     Q    L    L                             P
    Y        Q                Q    L    L                           L
    P                        I         Q
    Q                                  Q
    L                                     L
    A
    P
    N
    E
    C
    V
    S   K
     A  •
        I M S P L Y P P L L S A I P A Q S C Q P F I T(NH2)
```